# FASCICULE DE BREVET EUROPEEN

(11) **EP 2 092 961 B1**
(45) Date de publication et mention de la délivrance du brevet: **24.07.2013**
(21) Numéro de dépôt: 09153336.4
(22) Date de dépôt: 20.02.2009
(51) Int. Cl.: A61Q 5/10, A61K 8/84

(54) **Utilisation de polymères cationiques particuliers comme agents anti-oxydants ou anti-radicalaires**
Verwendung spezieller kationischer Polymere als Antioxidanzien oder Mittel zur Bindung freier Radikale
Use of particular cationic polymers as anti-oxidant or anti-radical agents

(30) Priorité: 22.02.2008 FR 0851166
(43) Date de publication de la demande: 26.08.2009
(73) Titulaire: L'Oréal, 75008 Paris (FR)
(72) Inventeur: Ascione Jean-Marc, 75003, Paris (FR); Cannell, David W., New Jersey NJ 07060 (US)
(74) Mandataire: Fevrier, Murielle Françoise E.

(56) Documents cités:
- EP-A- 0 884 047
- EP-A- 1 762 223
- WO-A-2004/087086
- US-A- 4 422 853
- T.E. GOTTSCHALCK, G.N. MCEWEN, JR.: "International Cosmetic Ingredient Dictionary and Handbook" 2006, THE COSMETIC, TOILETRY AND FRAGRANCE ASSOCIATION , WASHINGTON, D.C. , XP002503860 ISBN 1-882621-36-0 11ième édition Vol. 1, page 991 * colonne 1 *
- T.E. GOTTSCHALCK, G.N. MCEWEN, JR.: "International Cosmetic Ingredient Dictionary and Handbook" 2006, THE COSMETIC, TOILETRY AND FRAGRANCE ASSOCIATION , WASHINGTON DC , XP002503861 SBN 1-882621-36-0 11ième édition Vol. 2, page 1845 * colonne 1 - colonne 2 *

## Description

La présente invention concerne l'utilisation de polymères cationiques particuliers comme agents anti-oxydants ou anti-radicalaires (ou anti radicaux libres), notamment dans une composition cosmétique, en particulier une composition de coloration des cheveux.

Il est connu d'utiliser des agents antioxydants et/ou anti radicalaires dans les compositions cosmétiques pour lutter contre la dégradation des compositions elles mêmes ou des substrats sur lesquels elles sont appliquées. En particulier, il est connu d'utiliser des agents antioxydants ou anti radicaux libres pour diminuer la dégradation des cheveux lors de l'application de compositions capillaires.

Par exemple, la demande de brevet WO 2004/087086 décrit une composition cosmétique comprenant un composé anti-radicalaire présentant une constante de diffusion inférieure ou égale à 10⁻¹⁴ m²/s. De très nombreux composés anti radicalaires répondant à cette définition sont cités.

Les composés anti oxydants ou anti radicalaires utilisés jusqu'à présent sont des produits parfois peu stables dans les compositions cosmétiques ou sur les substrats sur lesquels ils sont appliqués. Ces produits présentent par ailleurs le plus souvent peu d'affinité pour ces substrats, notamment pour les matières kératiniques telles que les cheveux. Ils sont alors facilement éliminés ce qui réduits leur capacité à limiter la dégradation du substrat. Leur impact cosmétique est donc le plus souvent médiocre voire parfois défavorable.

Enfin, leur efficacité est souvent limitée, en particulier dans des compositions oxydantes.

Le but de la présente invention est de mettre à disposition de nouveaux systèmes antioxydants ou anti-radicaux libres ne présentant pas les inconvénients de la technique antérieure. En particulier, le but de l'invention est de fournir des antioxydants ou anti radicalaires très efficaces même en milieu oxydant, faciles à mettre en oeuvre dans les compositions cosmétiques, stables, présentant une bonne affinité pour les substrats sur lesquels ils sont appliqués tels que les cheveux, ayant un bon effet cosmétique .

Ainsi, la présente invention a pour objet l'utilisation d'un polycondensat de diammonium quaternaire comprenant des motifs récurrents répondant à la formule 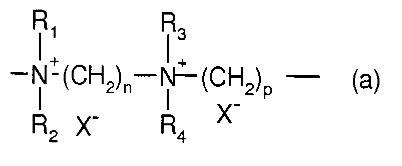 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 6 environ et, X⁻ est un anion dérivé d'un acide minéral ou organique.

Cette utilisation permet d'obtenir une moindre altération des substrats sur lesquels est appliqué le polycondensat pendant ou après l'application.Ce meilleur respect de l'intégrité des substrats se traduit le plus souvent par un meilleur état cosmétique.

Les substrats sont essentiellement les matières kératiniques et plus particulièrement les fibres kératiniques. Les cheveux sont encore plus particulièrement concernés.

La présente inventin concerne en particulier lutilisation d'un polycondensate de formule (a) dans une composition de coloration des cheveux comprenant au moins un colorant d'oxydation.

Selon un mode de réalisation particulier, l'invention concerne l'utilisation d'un polycondensat ammonium quaterniaire de formule (a) en tant qu'anti-radicalaire ou antioxydant dans une composition de coloration des cheveux comprenant au moins un colorant d'oxydation.

Le polycondensat de formule (a) est générallement appliquée dans une composition cosmétique. Une telles composition cosmétique comprend un ou plusieurs polycondensats de diammonium quaternaire particuliers.Ces polycondensats sont décrits ci-dessus.

,

Un composé de formule (a) particulièrement préféré est celui pour lequel R₁, R₂, R₃ et R₄ représentent un groupe méthyle et n = 3, p= 6 et X = Cl, c'est-à-dire le chlorure d'hexadimethrine dénommé "Hexadimethrine chloride" selon la nomenclature INCI (CTFA).

Selon un mode de réalisation particulier, le polycondensat utile dans la présente invention présente une charge cationique supérieure à 5 meq/g, de préférence supérieure à 6 meq/g.Cette densité de charge peut se déterminer soit par calcul à partir de la structure du polymère soit expérimentalement par la méthode Kjeldahl.

Selon un mode de réalisation, le polycondensat est mis en oeuvre dans une composition aqueuse, en particulier une composition cosmétique capillaire comprenant un ou plusieurs agents oxydants. De telles compositions capillaires sont généralement utiles pour la coloration ou la décoloration des cheveux.

A titre d'agents oxydants pouvant être utilisés, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases et leurs cofacteurs éventuels tels que l'acide urique pour les uricases. Le peroxyde d'hydrogène est particulièrement préféré.

Le pH de la composition est généralement compris entre les valeurs 2 et 14. I1 est de préférence compris entre 5 et 13.

Parmi les agents acidifiants on peut citer, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Selon une variante, la composition cosmétique présente un pH alcalin entre 7 et 14, de préférence entre 8 et 12. A titre d'agents alcalins pouvant être présents, on peut citer l'ammoniaque, les carbonates alcalins, le carbonate d'ammonium, les acides aminés et en particulier les acides aminés basiques tels que la lysine ou l'arginine, les alcanolamines telles que les mono-, di- et triéthanolamines ainsi que leurs dérivés, les hydroxydes de sodium ou de potassium et les composés de formule suivante : 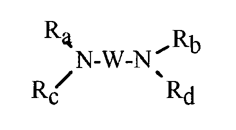 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

Le polycondensat est généralement présent dans la composition cosmétique dans des quantités comprises allant de 0,00001% à 10% en poids par rapport au poids de la composition.

La quantité d'eau présente dans la composition cosmétique est en général comprise entre 5 et 95 %, de préférence 20 et 90 %, mieux encore 30 et 80 %.

La composition peut se présenter sous des formes diverses, telles que sous forme de liquides, de crèmes, de gels, ou sous toute autre forme appropriée pour être appliquer sur les fibres kératiniques, et notamment des cheveux humains. La composition cosmétique comprend le polycondensat peut être une composition cosmétique de traitement des cheveux ou une composition de coloration.

L'invention permet de lutter efficacement contre l'effet destructeur des radicaux hydroxyles OH°. Cette action sur les radicaux hydroxyles est intéressante dans les milieux oxydants utilisés en coloration d'oxydation des cheveux car ces radicaux sont souvent responsables de la dégradation des fibres kératiniques. Cette utilisation permet notamment d'améliorer l'effet anti radicalaire et/ou antioxydant, lors du mélange de cette composition avec un agent oxydant, tel que le peroxyde d'hydrogène.

Lorsque la composition cosmétique utile dans la présente invention est une composition de coloration des cheveux, notamment une composition de coloration par oxydation des cheveux, elle comprend en général une ou plusieurs bases d'oxydation, et éventuellement un ou plusieurs coupleurs.

A titre de bases d'oxydation classiquement utilisées, on peut citer les ortho- et para-phénylènediamines, les bases doubles, les bis-phénylalkylènediamines, les ortho- et para- aminophénols, les bases hétérocycliques suivantes ainsi que leurs sels d'addition avec un acide. On peut citer :
(A) les paraphénylènediamines de formule (II) suivante et leurs sels d'addition avec un acide : 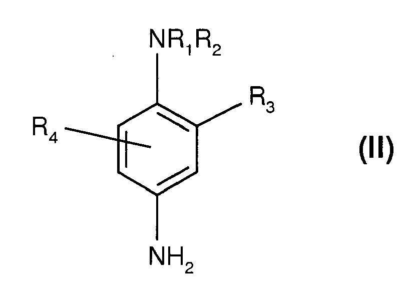
dans laquelle :
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthyl-paraphénylènediamine, la 2-fluoroparaphénylènediamine, la 2-isopropyl-paraphénylènediamine, la N-(β-hydroxypropyl)-paraphénylènediamine, la 2-hydroxyméthylparaphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (B) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales prêtes à l'emploi conformes à l'invention, on peut notamment citer les composés répondant à la formule (III) suivante, et leurs sels d'addition avec un acide : 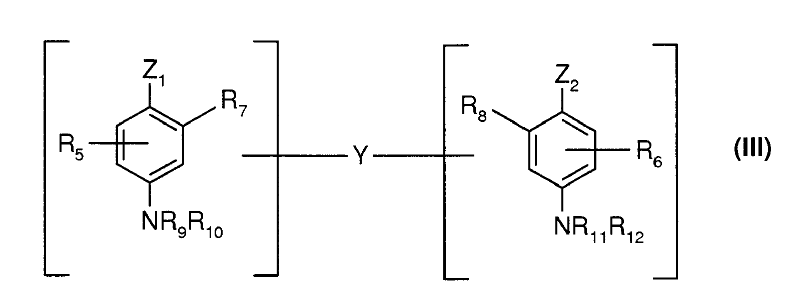 dans laquelle :
- Z₁ et Z₂, identiques ou différents, représentent un radical hydroxyle ou
- NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (III) ne comportent qu'un seul bras de liaison Y par molécule.
   Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (III) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (III), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (C) les para-aminophénols répondant à la formule (IV) suivante, et leurs sels d'addition avec un acide : 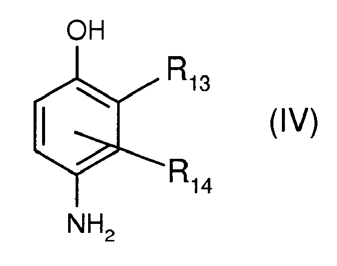
dans laquelle :
R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄. R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (D) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (E) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

A titre de dérivés pyrazoliques, on peut aussi citer les diamino-N,N-dihydropyrazopyrazolones et notamment celles décrites dans la demande FR 2 886 136 telles que les composés suivants et leurs sels d'addition.

Parmi ces composés, les préférés sont les :
2,3-Diamino-6,7-dihydro-1H,5H-pyrazolo[1,2-a]pyrazol-1-one,
2-Amino-3-éthylamino-6,7-dihydro-1H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)-6,7-dihydro-1H,5H-pyrazolo[1,2-a]pyrazol-1-one,
4,5-diamino-1,2-diméthyl-1,2-dihydro-pyrazol-3-one,
4,5-diamino-1,2-diéthyl-1,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one,

A titre de bases hétérocycliques, on utilisera préférentiellement le 4,5-diamino 1-(β-hydroxyéthyl) pyrazole, la 2,3-diamino-6,7-dihydro-1H,5H-pyrazolo[1,2-a]pyrazol-1-one et leurs sels d'addition.

Les ou les coupleurs utilisables sont ceux classiquement utilisés dans les compositions de teinture d'oxydation, c'est-à-dire les métaaminophénols, les méta-phénylènediamines, les métadiphénols, les naphtols, les coupleurs naphtaléniques et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, 1'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La ou les bases d'oxydation et le ou les coupleurs sont en général présentes en une quantité allant de 0,0005 à 12 % en poids, de préférence en une quantité allant de 0,01 à 8 % en poids, par rapport au poids total de la composition.

D'une manière générale, les sels d'addition avec un acide des bases d'oxydation et des coupleurs sont notamment choisis parmi les chlorhydrates, les bromhydrates, les sulfates et les tartrates, les lactates et les acétates.

La composition de coloration peut aussi être une composition de coloration directe éclaircissante. Cette composition peut alors contenir un ou plusieurs colorants directs pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Le ou les colorants directs représentent de préférence de 0,001 à 20% en poids environ du poids total de la composition prête à l'emploi et encore plus préférentiellement de 0,005 à 10% en poids environ

Selon une variante, la composition de coloration comprend en mélange une ou plusieurs bases d'oxydation, et un ou plusieurs colorants directs.

Le milieu approprié (ou support) de la composition utile à l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% en poids et, de préférence, entre environ 2 et 10% en poids par rapport au poids total de la composition.

La composition cosmétique utile dans l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères autres que les polycondensats de l'invention, des agents épaississants minéraux ou organiques, d'autres agents antioxydants ou anti-radicaux libres, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition utile dans l'invention peut être appliquée sur les fibres kératiniques telles que les cheveux.

Selon un procédé mettant en oeuvre les compositions décrites précédemment, on applique ces compositions sur les fibres, on rince éventuellement ; Lorsqu'il s'agit d'une composition de coloration, on applique sur les fibres pendant un temps suffisant pour développer la coloration désirée, puis on rince, on lave éventuellement au shampooing, on rince à nouveau et on sèche.

Les exemples qui suivent sont destinés à illustrer l'invention sans pour autant en limiter la portée.

### EXEMPLE

Méthode d'évaluation de l'activité antioxydante/antiradicalaire des polymères de l'invention vis-à-vis d'un stress oxydatif UVA induit sur kératinocytes.

La technique d'évaluation de la photo-protection est réalisée suivant une méthode bien connue (J. of Photochemistry and Photobiology B : Biology 57 (2000) 102-112 TOBI et al : Glutathione modulates the level of free radicals produced in UVA irradiated cells). Cette technique utilise une sonde fluorescente, marqueur du stress oxydatif global intracellulaire, la 2'-7'-Dichlorofluorescine Diacétate (DCFH-DA).

### PRINCIPE

L'utilisation de la DCFH-DA comme marqueur du stress oxydatif repose sur ses propriétés physico-chimiques. I1 s'agit d'une molécule apolaire et non ionique capable de diffuser à travers les membranes cellulaires. Une fois à l'intérieur de la cellule, la DCFH-DA va être hydrolysée par des estérases intracellulaires en un composé non fluorescent : la DCFH ou 2,7-dichlorofluorescine. En présence d'espèces activées de l'oxygène, la DCFH est rapidement oxydée en un composé hautement fluorescent : la DCF ou 2,7-dichlorofluorescéine.

### MODE OPERATOIRE

Trois composés ont été testés :l'hexadimethrine chloride (Mexomere PO de CHIMEX correspondant à un polycondensat de formule (I)), l'acide citrique et la glycine (0.0001%p/v pour le Mexomere PO 30microM pour l'acide citrique et la glycine.
1. Traitement des kératinocytes A confluence, les kératinocytes sont incubés en présence des composés à tester pendant 24 heures à 37°C, 5% de CO2, dans le milieu de culture, selon un effet dose (3 concentrations).
2. Incorporation de la DCFH-DA Les kératinocytes, prétraités sont rincés puis incubés en présence de DCFH-DA à l'obscurité.
3. Exposition aux UVA
   A l'issue de cette incubation, la solution de DCFH-DA est éliminée, les cellules sont ensuite exposées aux UVA(2J/cm2).
   Remarque : une plaque témoin non exposée est conservée à l'obscurité, à température ambiante.
4. Mesure de la fluorescence La fluorescence de la DCF est évaluée immédiatement après l'exposition aux UVA, par spectrofluorimétrie (excitation : 480nm ; émission : 530nm).
5. Résultats :
   - Le Mexomere PO présente une efficacité antioxydante, vis à vis du stress oxydatif UVA induit sur HaCaT, avec une protection d'environ 23% à 0.0001%(soit 0.08µM)
   - L'acide citrique et la glycine présentent une efficacité antioxydante avec un niveau de protection proche de 18 et 15% à 30 µM.
   - L'efficacité du Mexomere PO est supérieure à celle de la glycine ou de l'acide citrique.

### EXEMPLE DE COMPOSITION

On a préparé les deux compositions tinctoriales suivantes: (exprimé en grammes sauf autre indication)
Composition tinctoriale.

| | CONCENTRATION EN G |
|---|---|
| THIOLACTATE D'AMMONIUM EN SOLUTION AQUEUSE A 58 % (50 % EN ACIDE THIOLACTIQUE) | 0,80 |
| AMMONIAQUE ( CONCENTRATION DE REFERENCE 20% EN AMMONIAC) | 10 |
| MONOETHANOLAMINE PURE | 1,35 |
| SILICE PYROGENEE A CARACTERE HYDROPHOBE | 1,20 |
| 1-BETA-HYDROXYETHYLOXY-2,4-DIAMINO-BENZENE DICHLORHYDRATE | 0,15 |
| 2-METHYL-1,3-DIHYDROXYBENZENE (2-METHYLRESORCINOL) | 0,81 |
| OXYDE DE TITANE (ANATASE NON TRAITE) ENROBE DE POLY DIMETHYLSILOXANE (98/2) (Cl: 77891) | 0,15 |
| SULFATE DE N,N-BIS(2-HYDROXYETHYL)-P-PHENYLENEDIAMINE, 1 H2O | 0,21 |
| 1,3-DIHYDROXYBENZENE (RESORCINOL) | 1,29 |
| 1-HYDROXY-3-AMINO-BENZENE | 0,33 |
| 1,4-DIAMINO-BENZENE | 1,84 |
| DISTEARATE DE GLYCOL | 2 |
| ALCOOL CETYLSTEARYLIQUE (C16/C18 50/50) | 11,50 |
| PARFUM | 0,60 |
| POLYCONDENSAT TETRAMETHYL HEXAMETHYLENEDIAMINE / DICHLORO 1,3-PROPYLENE EN SOLUTION AQUEUSE | 4 |
| POLYMERE CARBOXYVINYLIQUE SYNTHETISE DANS LE MELANGE ACETATE D'ETHYLE/CYCLOHEXANE | 0,60 |
| PROPYLENE GLYCOL | 7 |
| ACIDE LAURIQUE NATUREL | 3 |
| ALCOOL LAURIQUE OXYETHYLENE (12 OE) | 7 |
| ALCOOL DECYLIQUE OXYETHYLENE (3 OE) | 10 |
| ALCOOL OLEOCETYLIQUE OXYETHYLENE (30 OE) | 4 |
| EAU DESIONISEE | QSP 100G |

Cette composition est mélangée poids pour poids avec un agent oxydant à pH 2,3 comprenant 9% de peroxyde d'hydrogène. On applique 30 min le mélange obtenu sur des cheveux gris à 90% de blancs. Après rinçage et séchage les cheveux sont teints dans une nuance chatain. Les cheveux sont doux et peu dégradés.

## Revendications

1. Utilisation pour le traitement des cheveux d'un polycondensat de diammonium quaternaire comprenant des motifs récurrents répondant à la formule (a) suivante en tant qu'agent anti radicalaire ou autioxydant: 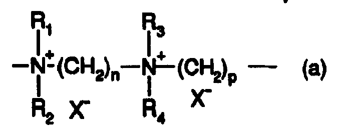 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 6 environ et, X⁻ est un anion dérivé d'un acide minéral ou organique.

2. Utilisation selon la revendication 1 dans laquelle le polycondensat de diammonium quaternaire particulier est le chlorure d'hexadimethrine pour lequel R₁, R₂, R₃ et R₄ représentent un groupe méthyle et n = 3, p= 6 et X = C1.

3. Utilisation selon l'une des revendications précédentes dans une composition cosmétique aqueuse.

4. Utilisation selon la revendication 3 dans laquelle le(s) polycondensat(s) de diammonium quaternaire de formule (a) sont présents chacun en une quantité allant de 0.00001% à 10% en poids par rapport au poids total de la composition

5. Utilisation selon la revendication 3 **caractérisée en ce que** la composition comprend un ou plusieurs agents oxydants.

6. Utilisation selon la revendication 3 dans laquelle l'agent oxydant est choisi parmi le peroxyde d'hydrogène, le peroxyde d'urée, les bromates de métaux alcalins, les persels, les peracides et les enzymes oxydases.

7. Utilisation selon l'une quelconque des revendications précédente 3 à 6 dans laquelle la composition comprend une ou plusieurs bases d'oxydation choisies parmi les paraphénylènediamines, les bis-phénytalkylènediainince, les paraaminophénols, les ortho-aminophénols, les bases hétérocycliques et leurs sels d'addition avec un acide, et éventuellement un coupleur choisi parmi les métaphénylènediamines, les métaaminophénols, les métadiphénols, les coupleurs naphtaléniques, les coupleurs hétérocycliques et lours sels d'addition avec un acide.

8. Utilisation selon l'une quelconque des revendications précèdentes 3 à 7 dans laquelle la composition comprend un ou plusieurs colorants directs.

9. Utilisation selon l'une quelconque des revendications précédentes 3 à 8 dans laquelle la composition présente un pH compris entre 7 et 14, de préférence entre 8 et 12.

## Patentansprüche

1. Verwendung eines quat.-Diammonium-Polykondensats, umfassend Wiederholungseinheiten der folgenden Formel (a), als Mittel gegen Radikale oder Antioxidans zur Behandlung der Haare: 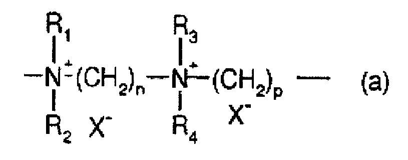 worin R₁, R₂, R₃ und R₄ gleich oder verschieden sind und für eine Alkyl- oder Hydroxyalkylgruppe mit ungefähr 1 bis 4 Kohlenstoffatomen stehen, n und p für ganze Zahlen im Bereich von ungefähr 2 bis 6 stehen und X⁻ für ein von einer anorganischen oder organischen Säure abgeleitetes Anion steht.

2. Verwendung nach Anspruch 1, wobei es sich bei dem speziellen quat.-Diammonium-Polykondensat um Hexadimethrinchlorid handelt, für das R₁, R₂, R₃ und R₄ für eine Methylgruppe stehen und n = 3, p = 6 und X = Cl.

3. Verwendung nach einem der vorhergehenden Ansprüche in einer wässrigen kosmetischen Zusammensetzung.

4. Verwendung nach Anspruch 3, wobei das quat.-Diammonium-Polykondensat bzw. die quat.-Diammonium-Polykondensate der Formel (a) in einer Menge im Bereich von 0,00001 bis 10 Gew.-%, bezogen auf das Gesamtgewicht der Zusammensetzung, vorliegt bzw. vorliegen.

5. Verwendung nach Anspruch 3, **dadurch gekennzeichnet, dass** die Zusammensetzung ein oder mehrere Oxidationsmittel umfasst.

6. Verwendung nach Anspruch 3, wobei das Oxidationsmittel aus Wasserstoffperoxid, Harnstoffperoxid, Alkalimetallbromaten, Persalzen, Persäuren und Oxidaseenzymen ausgewählt ist.

7. Verwendung nach einem der vorhergehenden Ansprüche 3 bis 6, wobei die Zusammensetzung eine oder mehrere Oxidationsbasen, die aus paraPhenylendiaminen, Bisphenylalkylendiaminen, para-Aminophenolen, ortho-Aminophenolen, heterocyclischen Basen, und Additionssalzen davon mit einer Säure ausgewählt sind, und gegebenenfalls einen Kuppler, der aus meta-Phenylendiaminen, meta-Aminophenolen, meta-Diphenolen, Kupplern auf Naphthalinbasis, heterocyclischen Kupplern sowie Additionssalzen davon mit einer Säure ausgewählt ist, umfasst.

8. Verwendung nach einem der vorhergehenden Ansprüche 3 bis 7, wobei die Zusammensetzung einen oder mehrere Direktfarbstoffe umfasst.

9. Verwendung nach einem der vorhergehenden Ansprüche 3 bis 8, wobei die Zusammensetzung einen pH-Wert zwischen 7 und 14, vorzugsweise zwischen 8 und 12, aufweist.

## Claims

1. Use, for treating hair, of a quaternary diammonium polycondensate comprising repeat units corresponding to the formula (a) below as free-radical scavenger or antioxidant: 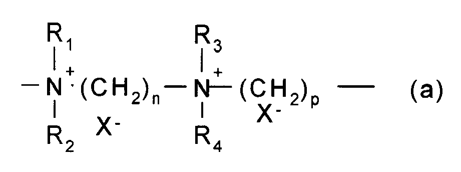 in which R₁, R₂, R₃ and R₄, which are identical or different, denote an alkyl or hydroxyalkyl group having from 1 to 4 carbon atoms approximately, 1 n and p are integers that vary from 2 to 6 approximately and X⁻ is an anion derived from a mineral or organic acid.

2. Use according to Claim 1, in which the particular quaternary diammonium polycondensate is hexadimethrine chloride for which R₁, R₂, R₃ and R₄ represent a methyl group and n = 3, p = 6 and X = Cl.

3. Use according to either of the preceding claims, in an aqueous cosmetic composition.

4. Use according to Claim 3, in which the quaternary diammonium polycondensate(s) of formula (a) are each present in an amount ranging from 0.00001% to 10% by weight relative to the total weight of the composition.

5. Use according to Claim 3, **characterized in that** the composition comprises one or more oxidizing agents.

6. Use according to Claim 3, in which the oxidizing agent is chosen from hydrogen peroxide, urea peroxide, alkali metal bromates, persalts, peracids and oxidase enzymes.

7. Use according to any one of the preceding Claims 3 to 6, in which the composition comprises one or more oxidation bases chosen from bisphenylalkylenediamines, *para*-phenylenediamines, *ortho*-aminophenols, *para*aminophenols, and heterocyclic bases, the addition salts thereof with an acid, and optionally a coupler chosen from *meta*-phenylenediamines, *meta*-aminophenols, *meta*-diphenols, naphthalenic couplers, heterocyclic couplers and the addition salts thereof with an acid.

8. Use according to any one of the preceding Claims 3 to 7, in which the composition comprises one or more direct dyes.

9. Use according to any one of the preceding Claims 3 to 8, in which the composition has a pH between 7 and 14, preferably between 8 and 12.
